Europäisches Patentamt

(19) European Patent Office

Office européen des brevets

(11) Numéro de publication: **0 224 533**
**B1**

(12) **FASCICULE DE BREVET EUROPÉEN**

(45) Date de publication du fascicule du brevet:
**21.03.90**

(21) Numéro de dépôt: **86903416.5**

(22) Date de dépôt: **30.05.86**

(86) Numéro de dépôt international:
**PCT/FR 86/00186**

(87) Numéro de publication international:
**WO 86/06953 (04.12.86 Gazette 86/26)**

(51) Int. Cl. ⁵: **A 61 B 17/22**

(54) CATHETER A FIBRE OPTIQUE POUR REALISER UNE DESOBSTRUCTION PAR L'INTERMEDIAIRE DE L'ENERGIE LUMINEUSE D'UN LASER.

(30) Priorité: **31.05.85 FR 8508582**

(43) Date de publication de la demande:
**10.06.87 Bulletin 87/24**

(45) Mention de la délivrance du brevet:
**21.03.90 Bulletin 90/12**

(84) Etats contractants désignés:
**AT BE CH DE GB IT LI LU NL SE**

(56) Documents cité:
**WO-A-84/04879
DE-A-3 212 180
FR-A-2 480 107
FR-A-2 482 304
US-A-3 858 577**

(73) Titulaire: LEFEBVRE, Jean-Marie
219, boulevard de la Liberté
F-59800 Lille (FR)

(72) Inventeur: LEFEBVRE, Jean-Marie
219, boulevard de la Liberté
F-59800 Lille (FR)

(74) Mandataire: Lepage, Jean-Pierre
Cabinet Lepage & Aubertin Innovations et Prestations
23/25, rue Nicolas Leblanc B.P. 1069
F-59011 Lille Cédex 1 (Nord) (FR)

LIBERGRAF, STOCKHOLM 1990

## Description

L'invention est relative à un cathéter à fibre optique, notamment destiné à conduire une énergie, telle que l'énergie lumineuse d'un laser, dans un vaisseau sanguin tel qu'une artère pour sa désobstruction par voie transluminale percutanée. Elle trouvera notamment son application dans le domaine médical pour le traitement de maladies telles que l'artérite.

L'artérite est un rétrécissement des vaisseaux d'origine athéromateuse pouvant l'obstruer partiellement, il s'agit dans ce cas d'une sténose, ou complètement, il s'agit d'une thrombose.

Dans de nombreux cas d'artérite, il est connu de réaliser une désobstruction artérielle par voie percutanée au moyen d'un cathéter muni d'un ballonnet; cette technique est généralement appelé angioplastie transluminale percutanée par ballon.

Pour ce, on introduit par ponction de l'artère un cathéter muni d'un ballonnet gonflable permettant de tasser l'athérome, et de faire disparaître le rétrécissement ou l'obstruction des vaisseaux.

Cette technique, bien que donnant de bons résultats, comporte néanmoins un inconvénient majeur qui réside dans le fait qu'il faut au préalable traverser l'obstacle; ce qui est,dans un certain nombre de cas, difficile, voire même impossible. En effet, bien souvent le pertuis de la sténose est très fin et difficile à trouver ou encore la thrombose difficile à perforer.

Plus récemment, il est apparu une nouvelle technique pour désobstruer des vaisseaux; cette technique utilise l'énergie d'un rayon laser pour détruire par vaporisation la lésion ou, du moins réaliser un petit chenal dans lequel on pourra introduire dans un second temps un cathéter à ballon.

Cette technique de traitement a nécessité la réalisation de cathéters à fibre optique permettant l'utilisation du rayon laser pour traiter localement l'artère. Néanmoins, pour que cette technique de traitement soit efficace et sans danger, il est nécessaire de mettre au point des cathéters de technique simple, d'utilisation facile et sûre.

En effet, l'énergie laser doit être amenée au contact de la lésion à traiter le plus précisément possible. Dans l'état actuel de la technologique, seule la fibre optique autorise ce transport d'énergie de manière facilement maîtrisable. En effet, grâce à la souplesse des fibres optiques, on peut amener son extrémité au contact de la lésion dans le vaisseau et ainsi véhiculer l'énergie laser jusqu'la zone à traiter.

Le but de la technique de désobstruction artérielle par énergie laser est donc de véhiculer une énergie suffisante pour détruire la lésion athéromateuse. Pour cela, il est nécessaire d'utiliser une énergie élevée telle que notamment l'énergie d'un laser du type YAG.

Toutefois, des précautions doivent être prises afin de ne détruire que la lésion pathologique sans risque de léser la paroi artérielle saine qui pourrait avoir des conséquences néfastes telles que notamment perforation avec hémorragies ou lésion pouvant être responsable d'une réthrombose secondaire.

En résume, bien que présentant certains avantages, la désobstruction artérielle utilisant l'énergie laser impose certaines contraintes techniques suivantes:

- faire cheminer la fibre optique à l'intérieur du vaisseau pour l'amener au contact de la lésion,
- repérer l'extrémité de la fibre pour l'amener au contact de la lésion,
- véhiculer une énergie suffisante pour détruire la lésion,
- veiller à ce que cette énergie ne puisse pas détériorer le cathéter et la fibre optique,
- laver l'extrémité de la fibre optique et évacuer rapidement les gaz formés lors du traitement,
- détruire la lésion sans léser la paroi artérielle,
- maniement facile, durant le traitement, du cathéter et de la fibre.

Actuellement, il existe un mode de réalisation de cathéters à fibres optiques permettant la mise en oeuvre de la technique de désobstruction artielle par laser. Bien que donnant quelques résultats satisfaisants, ces cathéters ne donnent pas entière satisfaction au praticien qui les utilise.

Cette réalisation est en fait un cathéter à ballonnet classique, déjà utilisé dans la technique d'angioplastie transluminale percutanée par ballon auquel on adjoint une fibre optique.

En effet, ce cathéter comporte un tube principal muni d'une extrémité proximale et d'une extrémité distale. Il porte dans sa zone distale un ballonnet gonflable et présente un conduit intérieur.

Ce cathéter est introduit par voie percutanée selon les techniques classiques au moyen d'un introducteur percutanée, et il chemine et descend dans l'artère, aidé éventuellement par l'intermédiaire d'un mandrin coaxial introduit dans le conduit intérieur du cathéter. Ainsi, l'extrémité du cathéter peut être amenée jusqu'au contact de la lésion.

Ensuite le mandrin est retiré pour laisser place à une fibre optique qui est glissée à l'intérieur du cathéter. Lorsque la fibre est en place, le ballonnet est gonflé légèrement de manière à rendre le cathéter parfaitement coaxial et en position centrale par rapport à la lumière de l'artère de manière à pouvoir effectuer un tir coaxial d'énergie laser.

Par ailleurs, sur le conduit intérieur du cathéter conduisant la fibre optique, on branche un système de perfusion permettant une irrigation à l'extrémité distale refroidissant le système évitant un échauffement au niveau de l'extrémité de la fibre.

Ce type de réalisation de cathéter à fibre optique permet de répondre à certaines contraintes techniques imposées pré-citées, à savoir le cheminement du cathéter, la précision du tir co-axial, le refroidissement pour éviter la détérioration du cathéter.

Néanmoins, il ne permet pas de répondre à toutes ces contraintes et de ce fait présente des inconvénients pour le praticien l'utilisant.

En effet, le cathéter est muni d'un conduit central acceptant la fibre optique et simultanément le liquide de refroidissement et de lavage. Comme ce liquide est assez visqueux, il faut que le conduit interne soit suffisamment large et de ce fait, la fibre optique est mal guidée, rendant le tir d'énergie laser non précis.

Par ailleurs, comme la fibre optique est de par nature radio-transparente, lors de la visualisation radiologique du cathéter, on n'aura aucune information sur la position de l'extrémité de la fibre optique. Ce positionnement doit alors être fait mécaniquement c'est-à-dire par différences de longueurs entre le cathéter et la fibre optique.

Enfin, la présence du ballonnet augmente la grosseur du cathéter à introduire ce qui est pénalisant pour son introduction dans de petits vaisseaux et diminue l'efficacité du lavage et du refroidissement.

Cela étant, dans un autre domaine, il est connu du document FR-A-2 480 107 un coagulateur endoscopique à fibre laser, constitué d'un tube principal dans lequel sont prévus deux conduits, présentant des axes longitudinaux sensiblement parallèles à l'axe du tube principal, destinés à recevoir d'une part une fibre optique permettant de transporter l'énergie laser pour irradier une zone et d'autre part un faisceau de fibres optiques permettant de délivrer une image.

Cependant, le problème posé par ce document et les buts recherchés sont différents de celui de la présente invention et ne permettent pas de solutionner les inconvénients des systèmes connus en matière de cathéters de désobstruction de vaisseaux.

Le but de la présente invention est de proposer un cathéter de fibre optique qui puisse pallier à ces différents inconvénients afin de faciliter l'intervention du praticien lors de la désobstruction d'un vaisseau sanguin par voie transluminale percutanée.

Un des buts de la présente invention est de proposer un cathéter à fibre optique permettant de dissocier les fonctions, c'est-à-dire le support de la fibre optique et la conduite de la solution de refroidissement et de lavage. Ainsi, on facilite l'intervention du praticien grâce à une meilleure manipulation puis on augmente l'efficacité du traitement par un meilleur refroidissement et lavage.

Un des buts de la présente invention est de proposer un cathéter à fibre optique dont l'extrémité distale de la fibre optique puisse facilement être repérée radiologiquement lors de l'intervention.

Un autre but de la présente invention est de proposer un cathéter à fibre optique qui de par sa constitution permette de diminuer sa grosseur afin de faciliter notamment l'évacuation des gaz formés lors de la vaporisation de la lésion, et de cheminer dans des vaisseaux plus petits.

Un autre but de la présente invention est de proposer un cathéter à fibre optique qui ne comporte pas de ballonnet gonflable mais qui permette de réaliser des tirs d'énergie laser de bonnes précisions.

Un autre but de la présente invention est de proposer un cathéter à fibre optique qui permette de réaliser des tirs d'énergie laser non coaxialement au vaisseau afin de le désobstruer plus efficacement.

D'autres buts et avantages de la présente invention apparaîtront au cours de la description suivante qui n'est cependant donnée qu'à titre indicatif et qui n'a pas pour but de la limiter.

Selon la présente invention, le cathéter à fibre optique pour réaliser une désobstruction par l'intermédiaire de l'énergie lumineuse à laser, telle qu'une désobstruction d'un vaisseau sanguin par voie transluminale percutanée, comportant:

- un tube principal muni d'une extrémité proximale et d'une extrémité distale, apte à être introduit dans le dit vaisseau sanguin,
- le dit tube principal présentant au moins un premier conduit, muni d'un premier orifice de sortie à l'extrémité distale,
- une fibre optique, apte à véhiculer et à conduire l'énergie laser de l'extrémité proximale vers l'extrémité distale, placée dans le dit premier conduit, est caractérisé par le fait qu'il comporte un deuxième conduit muni d'un deuxième orifice de sortie à l'extrémité distale tel que
- le dit deuxième conduit soit apte à recevoir un mandrin semi-rigide pour faciliter l'introduction percutanée du cathéter, et son cheminement dans le vaisseau sanguin, et/ou à faire circuler une solution de refroidissement et de lavage, pour d'une part, irriguer l'extrémité distale du cathéter, et pour d'autre part, laver la zone du vaisseau sanguin traité,
- les dits premier et deuxième conduits présentent des axes longitudinaux sensiblement parallèles à l'axe du tube principal et tels que l'un ne soit pas contenu dans l'autre,

et en ce que la fibre optique est immobilisée en translation dans le dit premier conduit pour la rendre solidaire du cathéter, afin de faciliter son positionnement et son repérage interne dans le vaisseau, et débouche au niveau du dit premier orifice de sortie, telle que le dit premier orifice soit excentré par rapport au tube principal afin que, par rotation du cathéter, on puisse réaliser, par tirs laser successifs, la formation de plusieurs trous pour déboucher le dit vaisseau sanguin, sur une section pouvant atteindre jusque 5 mm$^2$.

L'invention sera mieux comprise si l'on se réfère à la description ci-dessous, ainsi qu'aux dessins en annexe qui en font partie intégrante.

La figure 1 représente le cathéter à fibre optique réalisé selon la présente invention.

La figure 2 représente en coupe l'extrémité distale du cathéter représenté a la figure 1.

La figure 3 représente une coupe selon l'axe III – III de la figure 2.

La figure 4 représente une vue en coupe selon l'axe IV – IV de la figure 2 montrant la désobstruction d'un vaisseau sanguin.

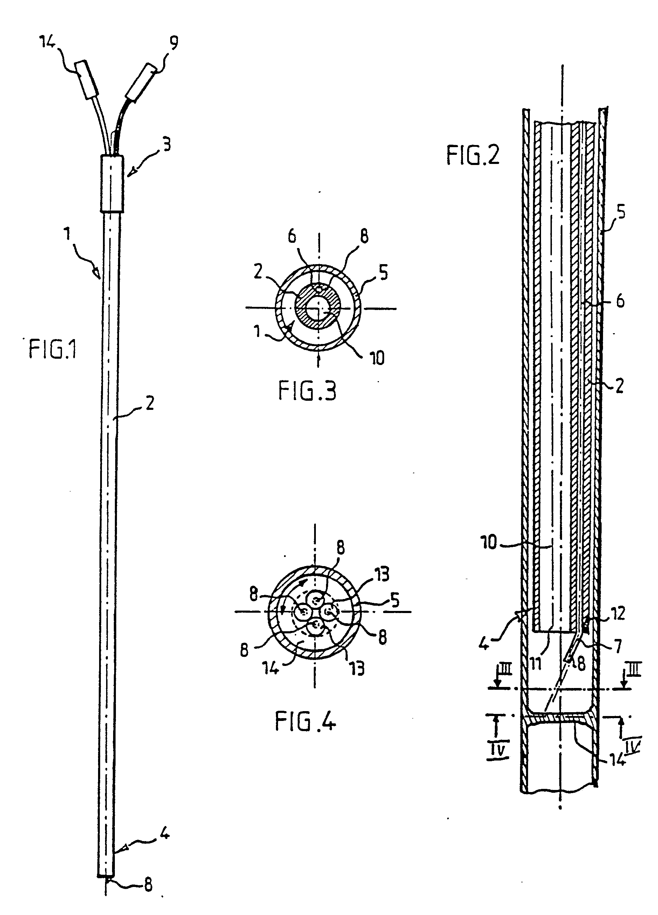

La figure 1 montre un cathéter 1 à fibre optique selon la présente invention, notamment destiné à conduire une énergie, telle que l'énergie lumineuse à laser, dans un vaisseau sanguin, tel qu'une artère, pour sa désobstruction par voie transluminale percutanée. Ce cathéter à fibre optique sera notamment utilisé par le praticien lors du traitement d'artérites.

Le cathéter à fibre optique 1 comporte un tube principal 2 muni d'une extrémité proximale 3 et d'une extrémité distale 4.

Plus précisément, comme le montrent les figures 2 et 3 le tube présente au moins un premier conduit 6 muni d'un premier orifice de sortie 7 à l'extrémité distale 4. Dans ce conduit 6 est placée une fibre optique 8.

Cette fibre optique sera notamment apte à véhiculer l'énergie lumineuse d'un laser. Elle sera raccordée à un générateur laser par une connexion 9 prévue à cet effet dans la zone proximale 3 du cathéter.

Selon l'invention, la fibre optique 8 est préalablement immobilisée en translation dans le premier conduit 6 pour la rendre solidaire du cathéter. On pourra notamment prévoir au niveau de la connexion 9 dans la zone proximale 3 un dispositif d'immobilisation tel que notamment une vis de pincement pour empêcher les mouvements relatifs de la fibre optique 8 par rapport au tube principal 2.

De ce fait, d'une part comme la fibre optique 8 est solidaire du tube 2, et d'autre part en réalisant le tube principal 2 du cathéter à partir d'un matériau inerte semi-rigide radio-opaque, on facilitera le positionnement et le repérage interne dans le vaisseau 5 de la fibre optique et plus particulièrement de son extrémité.

En effet, lors de la mise en oeuvre de la technique de désobstruction par laser, on contrôle en permanence les mouvements internes du cathéter par un contrôle radiologique et on pourra détecter l'extrémité du cathéter et en déduire de ce fait la position de l'extrémité de la fibre optique 8 par rapport à la lésion artérielle à traiter 14.

Il est à noter que dans un mode de réalisation préférentiel, la fibre optique présente un diamètre compris entre 50 et 500 microns. En outre, on fait dépasser la fibre optique de l'extrémité distale 4 du tube 2 d'une valeur comprise entre 1 à 10 mm.

Par ailleurs, selon la présente invention, le cathéter à fibre optique 1 comporte un deuxième conduite 10 muni d'un deuxième orifice de sortie 11 à l'extrémité distale 4. Ce deuxième conduit sera notamment apte à véhiculer un mandrin, non représenté sur les figures, facilitant l'introduction percutanée du cathéter 1 et son cheminement dans l'artère et/ou à véhiculer une solution de refroidissement et de lavage de la zone artérielle traitée.

Il est à noter que le mandrin sera notamment constitué par un style métallique semi-rigide de réalisation connu de l'Homme de l'Art. Par ailleurs, la solution de lavage aura avantageusement un certain pouvoir d'opacification pour faciliter le contrôle radiologique lors de l'intervention du praticien.

Selon la présente invention, les deux axes des conduits 6 et 10 sont sensiblement parallèles à l'axe du tube principal 2.

Le diamètre intérieur du conduit 10 sera prévu tel qu'il autorise un débit important de la solution de refroidissement et de lavage afin de permettre par un bon refroidissement d'éviter notamment la fusion de la fibre optique ou du cathéter et d'autre part de permettre par un lavage efficace l'élimination des gaz formés par la vaporisation de la lésion.

Il est à remarquer que le cathéter ne présentant pas de ballonnet de gonflage, l'évacuation des gaz formés en est facilitée. Néanmoins, compte tenu de l'absence de ce ballonnet, il est nécessaire de revoir le principe de l'axe de tir et d'imaginer un autre système, permettant un tir laser avec sécurité que ce soit dans un segment d'artère rectiligne ou dans un segment d'artère courbe.

A cet égard, selon un mode préférentiel de l'invention dans la zone de l'extrémité distale 4 du tube principal 2, l'axe du premier conduit 6 dans lequel est placée la fibre optique 8, est incliné par rapport à l'axe du tube principal 2. Comme le montre la figure 2, ces deux axes convergent extérieurement à l'extrémité distale du cathéter 1, le premier orifice de sortie 7 étant notamment excentré par rapport au tube 2.

Il est à noter que l'on a obtenu de bons résultats avec une inclinaison du conduit 6 par rapport à l'axe principal du tube 2 comprise entre 1 et 15 degrés. En outre, on pourra réaliser des cathéters présentant des inclinaisons différentes dépendant de l'utilisation du cathéter; en effet, on utilisera des angulations faibles pour des vaisseaux rectilignes et des angulations plus fortes pour des vaisseaux courbes.

Afin de permettre un meilleur repérage de l'extrémité du cathéter 1 placé dans le vaisseau à traiter, selon un mode préférence de l'invention, le tube principal 2 inclut dans sa zone distale 4 une tige métallique 12. Cette tige sera avantageusement inclinée sensiblement parallèlement à la partie inclinée de la fibre optique 8 comme le montre notamment la figure 2.

Ainsi, le praticien, d'une part, grâce à la visualisation de l'extrémité du cathéter 11 solidaire de la fibre optique 8, et d'autre part par la visualisation de l'angulation formée par l'extrémité de la fibre optique par rapport à l'axe du cathéter 1, aura de bonnes informations, par le contrôle radiologique, sur la position de l'extrémité de la fibre par rapport à la lésion à traiter pour permettre un tir laser en toute sécurité sans risque de détériorer la paroi artérielle voisine de la lésion.

En outre, le cathéter de la présente invention, du fait de l'angulation de la fibre optique vers l'intérieur présente deux avantages appréciables pour le praticien.

Le premier, sur le plan de la sécurité, l'angulation de l'extrémité de la fibre optique 8 permet de diriger le tir laser vers l'intérieur du vaisseau et de ce fait diminue les risques de léser les parois artérielles.

Le deuxième avantage de l'angulation ainsi formée réside dans le fait qu'elle accroît l'efficacité de la désobstruction; en effet, grâce à cette disposition, la surface détruite peut être multipliée par quatre, voire huit en fonction de l'angle de tir, par rapport aux techniques classiques c'est-à-dire lorsque le tir est coaxialement maintenu par le ballonnet gonflable.

En effet, comme le montre la figure 4, par déplacement angulaire relatif de l'extrémité de la fibre optique 8, on peut réaliser plusieurs tirs successifs permettant d'atteindre la partie lésée en plusieurs endroits repérés 13 sur la figure.

Il est à noter que de bons résultats de désobstruction sont notamment obtenus en faisant quatre tirs successifs par rotation du cathéter afin de positionner l'extrémité de la fibre optique à 90, 180, 270 et 360 degrés.

Généralement, le trou réalisé par le tir laser présente un diamètre de deux à quatre fois le diamètre de la fibre, c'est-à-dire lorsqu'on utilise par exemple une fibre de 200 microns, on crée des trous d'environ 800 microns de diamètre. En utilisant la technique du cathéter à fibre optique et ballonnet gonflable, or parvient à faire au maximum des trous de l'ordre de 0,5 mm$^2$.

Avec la cathéter selon la présente invention, grâce à la perforation multi-trous, on arrive à déboucher l'artère jusqu'à une section d'environ 5 mm$^2$. Par ailleurs, lors de la mise en oeuvre de ce cathéter à fibre optique, on pourra jouer sur la puissance du tir laser en réglant notamment le générateur du laser afin de moduler les dimensions des trous selon les conditions de tir.

De plus, il est à remarquer que la technique d'utilisation du cathéter à fibre optique de la présente invention reprend en grande partie la technique utilisée jusqu'à ce jour pour les autres cathéters tout en la facilitant.

En effet, le malade est allongé sur une table radiologique munie d'un amplificateur de brillance. Après anesthésie locale, une ponction artérielle permet d'introduire un guide métallique sur lequel on glisse un introducteur percutané muni d'une gaine qui sera laissée en place dans le vaisseau et servira à introduire le cathéter de la présente invention. Comme dans la technique classique utilisant un cathéter a ballonnet, on introduit le cathéter dans la gaine de sorte qu'il chemine et descende dans l'artère jusqu'au contact de la lésion.

Afin d'aider au cheminement du cathéter dans le vaisseau, on place un mandrin coaxial introduit dans le deuxième conduit du cathéter de l'invention c'est-à-dire celui prévu pour véhiculer la solution de refroidissement et de lavage. Ainsi l'extrémité du cathéter peut être amenée paisiblement jusqu'au contact de la lésion.

Le mandrin pourra alors être retiré mais ceci est non impératif car on pourra, si le besoin est, le laisser en place. En effet, le deuxième conduit véhiculant la solution de refroidissement et de lavage est prévu tel que l'espace soit suffisant pour perfuser simultanément de manière efficace.

Il est à remarquer que grâce aux dispositions qui viennent d'être décrites et notamment par la dissociation des deux fonctions, à avoir support de la fibre optique et conduite de la solution de refroidissement et de lavage, on facilite d'une part, la manipulation du cathéter par le praticien et on augmente d'autre part, l'efficacité du refroidissement et du lavage.

La perfusion sera notamment autorisée grâce à un deuxième embout de raccordement 14 prévu dans la partie proximale du cathéter 1 permettant de le relier à un dispositif d'alimentation en solution.

Naturellement, le mode de réalisation qui vient d'être décrit n'est donné qu'à titre indicatif.

## Revendications

1. Cathéter à fibre optique (1) pour réaliser une désobstruction par l'intermédiaire de l'énergie lumineuse à laser, telle qu'une désobstruction d'un vaisseau sanguin par voie transluminale percutanée, comportant:

- un tube principal (2) muni d'une extrémité proximale (3) et d'une extrémité distale (4), apte à être introduit dans le dit vaisseau sanguin (5),
- le dit tube principal (2) présentant au moins un premier conduit (6), muni d'un premier orifice de sortie (7) à l'extrémité distale (4),
- une fibre optique (8), apte à véhiculer et à conduire l'énergie laser de l'extrémité proximale (3) vers l'extrémité distale (4), placée dans le dit premier conduit (6), caractérisé par le fait qu'il comporte un deuxième conduit (10) muni d'un deuxième orifice de sortie (11) à l'extrémité distale (4) tel que:
- le dit deuxième conduit (10) soit apte à recevoir un mandrin semi-rigide pour faciliter l'introduction percutanée du cathéter (1), et son cheminement dans le vaisseau sanguin (5), et/ou à faire circuler une solution de refroidissement et de lavage, pour d'une part, irriguer l'extrémité distale (4) du cathéter (1), et pour d'autre part, laver la zone du vaisseau sanguin traité (5),
- les dits premier et deuxième conduits (6, 10) présentent des axes longitudinaux sensiblement parallèles à l'axe du tube principal (2) et tels que l'un ne soit pas contenu dans l'autre,

et en ce que la fibre optique (8) est immobilisée en translation dans le dit premier conduit (6) pour la rendre solidaire du cathéter (1), afin de faciliter son positionnement et son repérage interne dans le vaisseau, et débouche au niveau du dit premier orifice de sortie (7), telle que le dit premier orifice (7) soit excentré par rapport au tube principal (2) afin que, par rotation du cathéter (1), on puisse réaliser, par tirs laser successifs, la formation de plusieurs trous (13) pour déboucher le dit vaisseau sanguin (5), sur une section pouvant atteindre jusque 5 mm².

2. Cathéter selon la revendication 1, caractérisé par le fait que la dite fibre optique (8), dans la zone de l'extrémité distale (4) du tube principal (2), présente son axe incliné par rapport à l'axe du tube principal (2), ces deux axes convergent extérieurement à l'extrémité distale du cathéter.

3. Cathéter à fibre optique selon la revendication 1, caractérisé par le fait que le tube principal (2) du cathéter (1), inclut dans sa zone distale (4) une tige métallique (12) permettant un repérage de l'extrémité placée dans le vaisseau.

4. Cathéter à fibre optique selon la revendication 3, caractérisé par le fait que la tige métallique (12) est inclinée sensiblement parallèlement à la partie inclinée de la fibre optique.

5. Cathéter à fibre optique selon la revendication 1, caractérisé par le fait que la fibre optique dépasse de l'extrémité distale (4) du tube (2) de 1 à 10 mm.

6. Cathéter à fibre optique selon la revendication 2, caractérisé par le fait que l'inclinaison de premier conduit (6) portant la fibre optique (8) par rapport à l'axe principale du tube (2) est compris entre 1 et 15 degrés.

7. Cathéter à fibre optique selon la revendication 6, caractérisé par le fait que l'inclinaison dépend de l'utilisation du cathéter (1), à savoir qu'elle est plus faible pour des vaisseaux rectilignes et plus forte pour des vaisseaux courbes.

8. Cathéter à fibre optique selon la revendication 1, caractérisé par le fait que la fibre optique présente un diamètre compris entre 50 et 500 microns.

**Patentansprüche**

1. Lichtleitfaser-Katheter (1) zur Beseitigung von Verstopfungen mittels Laser-Lichtenergie, wie beispielsweise die Beseitigung einer Verstopfung eines Blutgefäßes auf perkutanem, transluminalem Wege, aus:
- einem Hauptrohr (2), das mit einem proximalen Ende (3) und einem distalen Ende (4) versehen ist, und das sich zur Einführung in das besagte Blutgefäß (5) eignet,
- wobei das besagte Hauptrohr (2) mindestens eine erste Rohrleitung (6) aufweist, die an dem distalen Ende (4) mit einer ersten Ausgangsöffnung (7) versehen ist,
- einer Lichtleitfaser (8), die sich zur Beförderung und Leitung der Laserenergie von dem proximalen Ende (3) nach dem distalen Ende (4) eignet, und die in der besagten ersten Rohrleitung (6) angeordnet ist, *dadurch gekennzeichnet, daß* er eine zweite Rohrleitung (10) aufweist, die an dem distalen Ende (4) mit einer zweiten Ausgangsöffnung (11) versehen ist, wobei:
- die zweite Rohrleitung (10) geeignet ist, einen halbstarren Dorn aufzunehmen, um die perkutane Einführung des Katheters (1) und seine Weiterbewegung in dem Blutgefäß (5) zu erleichtern, und/oder eine Kühl- und Spüllösung umzuwälzen, um einerseits das distale Ende (4) des Katheters (1), und andererseits die Zone des behandelten Blutgefäßes (5) zu spülen,
- und die besagten Rohrleitungen (6, 10) im wesentlichen parallel zu der Achse des Hauptrohrs (2) verlaufende Längsachsen aufweisen, aber keine die andere umschließt,

und daß die Lichtleitfaser (8) in der besagten ersten Rohrleitung (6) seitlich blockiert ist, um sie mit dem Katheter (1) fest zu verbinden, so daß ihre Positionierung und ihre interne Lokalisierung in dem Gefäß erleichtert werden, und die besagte Lichtleitfaser (8) im Bereich der besagten ersten Ausgangsöffnung (7) nach außen geführt ist, wobei die besagte erste Öffnung (7) bezüglich des Hauptrohrs (2) exzentrisch angeordnet ist, damit durch Drehen des Katheters (1) und aufeinanderfolgende Laserschüsse mehrere Löcher (13) hervorgerufen werden können, um bei dem besagten Blutgefäß (5) über einen Querschnitt von bis zu 5 mm² die Verstopfung zu beseitigen.

2. Lichtleitfaser-Katheter gemäß Anspruch 1, *dadurch gekennzeichnet, daß* die Achse der besagten Lichtleitfaser (8) in der Zone des distalen Endes (4) des Hauptrohrs (2) bezüglich der Achse des Hauptrohrs (2) geneigt ist, wobei diese zwei Achsen außerhalb des distalen Endes des Katheters konvergieren.

3. Lichtleitfaser-Katheter gemäß Anspruch 1, *dadurch gekennzeichnet, daß* das Hauptrohr (2) des Katheters (1) in seiner distalen Zone (4) einen metallischen Stab (12) aufweist, mit dem das in dem Gefäß angeordnete Ende lokalisiert werden kann.

4. Lichtleitfaser-Katheter gemäß Anspruch 3, *dadurch gekennzeichnet, daß* der metallische Stab (12) im wesentlichen parallel zu dem geneigten Teil der Lichtleitfaser verläuft.

5. Lichtleitfaser-Katheter gemäß Anspruch 1, *dadurch gekennzeichnet, daß* die Lichtleitfaser um 1 bis 10 mm über das distale Ende (4) des Rohrs (2) hinausreicht.

6. Lichtleitfaser-Katheter gemäß Anspruch 2, *dadurch gekennzeichnet, daß* die Neigung der die Lichtleitfaser (8) enthaltenden ersten Rohrleitung (6) bezüglich der Hauptachse des Rohrs (2) zwischen 1 und 15 Grad liegt.

7. Lichtleitfaser-Katheter gemäß Anspruch 6, *dadurch gekennzeichnet*, daß die Neigung von der Verwendung des Katheters (1) abhängt, wobei sie bei geradlinigen Gefäßen schwächer, und bei gekrümmten Gefäßen stärker ist.

8. Lichtleitfaser-Katheter gemäß Anspruch 1, *dadurch gekennzeichnet*, daß die Lichtleitfaser einen Durchmesser zwischen 50 und 500 Mikron aufweist.

## Claims

1. An optical-fibre catheter (1) for effecting the removal of an obstruction through the agency of laser light energy, such as the removal of an obstruction from a bloodvessel by a percutaneous transluminal method, including:

   - a main tube (2) equipped with a proximal end (3) and a distal end (4) suitable for being introduced into the said bloodvessel (5);
   - the said main tube (2) exhibiting at least one first duct (6) equipped with a first outlet orifice (7) at the distal end (4);
   - an optical fibre (8) suitable for conveying and conducting laser energy from the proximal end (3) towards the distal end (4), placed in the said first duct (6); characterized by the fact that it includes a second duct (10) equipped with a second outlet orifice (11) at the distal end (4) and such that:
   - the said second duct (10) is suitable for receiving 3 semirigid mandrel for facilitating the percutaneous introduction of the catheter (1) and routing of it into the bloodvessel (5) and/or for making a cooling and washing solution circulate in order on the one hand to irrigate the distal end (4) of the catheter (1) and on the other hand to wash the bone of the bloodvessel (5) being treated;
   - the said first and second ducts (6, 10) exhibit longitudinal axes substantially parallel with the axis of the main tube (2) and are such that the one is not contained in the other;
   and in that the optical fibre (8) is fixed in

translation in the said first duct (6) so as to make it integral with the catheter (1) in order to facilitate its positioning and its internal registering in the vessel, and emerges at the level of the said first outlet orifice (7) so that the said first orifice (7) is offcentre with respect to the main tube (2) in order that by rotation of the catheter (1) one can by successive laser shots effect the formation of a number of holes (13) so as to unstop the said bloodvessel (5) over an area which may amount to as much as 5 mm².

2. A catheter as in Claim 1, characterized by the fact that in the zone of the distal end (4) of the main tube (2) the said optical fibre (8) presents its axis inclined with respect to the axis of the main tube (2) and these two axes converge outside the distal end of the catheter.

3. An optical-fibre catheter as in Claim 1, characterized by the fact that the main tube (2) of the catheter (1) encloses in its distal zone (4) a metal rod (12) enabling registering of the end located in the vessel.

4. An optical-fibre catheter as in Claim 3, characterized by the fact that the metal rod (12) is inclined substantially in parallel with the inclined portion of the optical fibre.

5. An optical-fibre catheter as in Claim 1, characterized by the fact that the optical fibre overhangs the distal end (4) of the tube (2) by from 1 to 10 mm.

6. An optical-fibre catheter as in Claim 2, characterized by the fact that the inclination with respect to the main axis of the tube (2), of the first duct (6) bearing the optical fibre (8), lies between 1 and 15 degrees.

7. An optical-fibre catheter as in Claim 6, characterized by the fact that the inclination depends upon the use of the catheter (1), viz., it is less for straight vessels and greater for curved vessels.

8. An optical fibre catheter as in Claim 1, characterized by the fact that the optical fibre exhibits a diameter lying between 50 and 500 microns.

FIG.1

FIG.2

FIG.3

FIG.4

14

9

3

1

2

4

8

6

8

2

5

1

10

5

6

2

10

4

11

12

7

8

14

III

III

IV

IV

8

8

8

13

5

8

14

13